# EUROPEAN PATENT APPLICATION

(11) **EP 2 730 284 A1**
(43) Date of publication of application: **14.05.2014**
(21) Application number: 12192192.8
(22) Date of filing: 12.11.2012
(51) Int. Cl.: A61K 31/565, A61P 15/18

(54) **Levonorgestrel-only-composition for optimized oral contraception with defined levonorgestrel content, dosage regimen and pharmaceutical preparation**

(71) Applicant: Naari AG, 4051 Basel (CH)
(72) Inventor: Oettel, Michael, 07743 Jena (DE); Kochhar, Prithi S., 4051 Basel (CH)
(74) Representative: Wablat Lange Karthaus

(57) **Abstract**

Based on specific experimental studies the invention relates to an optimized levonorgestrel-only-composition for improved continuous oral contraception with consequences for suitable pharmaceutical preparations for the same purpose and with defined levonorgestrel content .

The invention relates to a pharmaceutical composition for oral contraception for a woman of fertile age consisting of 60 to 100 µg of levonorgestrel and one or more pharmaceutical acceptable carriers and excipients. Furthermore, the invention relates to a pharmaceutical preparation for oral contraception consisting of a number of separately packed and individually removable daily dosage units intended for consecutive daily oral administration, each dosage unit consisting of 60 to 100 µg of levonorgestrel and one or more pharmaceutical acceptable carriers and excipients. Additionally, the invention relates to a dosage regimen, i.e. to the use of an oral dosage form consisting of 60 to 100 µg of levonorgestrel and one or more pharmaceutical acceptable carriers and excipients per dosage unit for oral contraception for a woman of fertile age by administering one dosage unit daily during the complete menstruation cycle.

## Description

Based on specific experimental studies the invention relates to a levonorgestrel-only-composition for continuous oral contraception with consequences for suitable pharmaceutical preparations for the same purpose and with defined levonorgestrel content.

Oral combined contraceptive pills (COCs) containing an estrogen- and a progestin-component are the most common method of contraception. These COCs show some well-documented side effects like thrombosis, myocardial infarction, stroke etc. when taken as oral contraceptives (Cackovic et al. 2008). However, these complications are associated with the use of COCs and not with the use of progestin-only pills (POPs; World Health Organization 1998). POPs are taken every day, with no placebo or pill-free interval and no change in pill formulation.

According to the US Medical Eligibility Criteria (MEC), many more conditions are considered relative (category 3) and absolute (category 4) contraindications for COCs compared to POPs (World Health Organization 1998; White et al. 2012). For example, hypertension, migraine headache with aura and smoking among women 35 years and older- conditions with the highest prevalence in clinical studies of reproductive-aged women - are not considered contraindications for POPs (Centers for Disease Control and Prevention, 1998; Shortridge and Miller, 2007; Grossman et al. 2008; Grossman et al. 2011). Most important: POPs carry minimal or no thrombotic risk (Cameron et al. 2011).

From this safety point of view, POPs must be the best choice for oral contraception. But, in contrast to COCs, POPs are rarely used (Hall et al. 2012). The aim of this invention is to change this situation.

The best known and investigated POP among others is the levonorgestrel-only-pill (LOP) with an oral dosage of only 30 µg per day and woman (e.g. Microlut^{®}, 28 mini^{®}). Whereas POP's are given daily without any break, the post-coital emergency pills contain 750 µg levonorgestrel (applied over two days) or 1.5 mg levonorgestrel (applied only once), for example Unofem^{®}.

The mode of action of this POP discussed in the scientific literature is multi-faceted and includes peripheral pre-ovulatory (cervical mucus hostility, sperm motility) as well as peripheral, post-ovulatory events (fertilization and transport of the eggs), whereas in most of the cases the ovulation is not inhibited.

Even if LOPs have been used for many years, they still have a limited acceptability by both women requesting contraception and doctors prescribing them. The reason for that is the elevated failure rate of the LOP (0.7 - 1.8/100 women per year). Based on the absence of anti-ovulatory activity, this failure rate is significantly higher than that of COCs (0.17 - 0.41/100 women per year; see: Oxford/FPA Study 1982). For this reason this pill is not in the first line of contraceptive methods.

The object of the present invention is be the provision of a new pharmaceutical preparation based on levonorgestrel only.

The object was solved by a pharmaceutical composition for oral contraception for a woman of fertile age consisting of 60 to 100 µg of levonorgestrel and one or more pharmaceutical acceptable carriers and excipients. Preferably, the pharmaceutical composition has 75-100 µg of levonorgestrel. No further active agent is present, for example, no further progestin and no estrogen. "Woman of fertile age" means also women not having reached the menopause, i.e. pre-menopausal women.

The term "pharmaceutical acceptable carriers and excipients" comprises excipients, but also diluents, flavoring or aromatising agents, stabilizers as well as formulation-promoting or formulation-providing additives, which are commonly used in the pharmaceutical practice.

The present invention also relates to a pharmaceutical preparation for oral contraception containing a number of separately packed and individually removable dosage units, each dosage unit consisting of 60 to 100 µg of levonorgestrel and one or more pharmaceutical acceptable carriers and excipients. Preferably, each dosage unit has 75-100 µg of levonorgestrel. The dosage units are intended for consecutive daily administration. A preferred embodiment contains 28 separately packed and individually removable dosage units. The dosage units are intended for consecutive daily oral administration for a period of at least 28 consecutive days, i.e. daily administration over the complete menstruation cycle. No dosage units having different composition and no placebos are used and no non-intake days are intended.

Furthermore, the present invention relates to a dosage regimen, i.e. the use of an oral dosage form consisting of 60 to 100 µg of levonorgestrel and one or more pharmaceutical acceptable carriers and excipients per dosage unit for oral contraception for a (pre-menopausal) woman of fertile age by administering one dosage unit daily during the complete menstruation cycle. Preferably, each dosage unit has 75-100 µg of levonorgestrel. The doses are taken continuously each day and no gap should exist between packs taken. No dosage units having different composition and no placebos are used and no non-intake days are intended. That is, new intake is started immediately after the end of the last cycle at the first day of the next cycle.

The pharmaceutical composition for oral contraception as well as the dosage unit can be in solid or liquid state, for example, tablets (with or without coating), capsules, pills or powder preparations. Liquid compositions can be for example solutions including sprays.

The aim of this invention was the design of a progestin-only contraceptive pill (POP) using levonorgestrel as progestational component. For this, specific animal studies were conducted investigating the dosage differences between peripheral and central contraceptive effects of levonorgestrel. It is well known, that on the field of sexual steroids the relations/proportions between the different directions of the pharmacodynamic profile of a given progestin like the anti-gonadotrophic action (e.g. inhibition of ovulation) vs. progestogenic action (e.g. pre- as well as post-ovulatory antifertility effects) in well designed animal studies reflect very well the dose-effect-relationships in humans and enable the determination of the human dose (Neumann et al. 1977; Sitruk-Ware 2006).Therefore, the results from our preclinical studies are leading to a new regimen for LOP which was unknown before.

As mentioned above, a levonorgestrel-containing POP with a daily dosage of only 0.03 mg (30 µg) is known for years. This dosage is only the half of the ovulation-inhibiting dose of 0.06 mg/day. It is well accepted, that the mode of action of this 0.030 mg-POP lies only in the stimulation of certain non-ovulatory, peripheral contraceptive effects, which are sufficiently for oral contraception. But as it shown in the practice, the contraceptive effect of the 30 µg-levonorgestrel-POP is insufficient due to the high failure rate.

Based on our suitable animal studies it was found that the dosage for central antigonadotrophic/antiovulatory effects of levonorgestrel is -depending from the chosen animal species- lower than that for all or complete peripheral contraceptive effects. These "inverse" relationships are not documented as yet for other progestins.

A levonorgestrel-only contraceptive having active ingredient content above the daily ovulation inhibition dose in humans (at least ≥0.06 mg/day and woman) shows better efficiency. Based on these unexpected findings it was realized that with higher daily dosages of levonorgestrel an additional contraceptive effect (surplus-effect) occurs by further peripheral, non- ovulation-related contraceptive effects not seen in lower dose ranges. This additional contraceptive effect of this high- dosage, levonorgestrel-containing POP should be also useful in such cases when women forget the daily intake and therefore ovulation has been occurred.

The actual scientific option is related to the following paradigm: Low-dose POPs like levonorgestrel 30 µg/day act mainly by influencing peripheral mechanisms and not by inhibition of ovulation. It is accepted and the actual doctrine means that the primary mechanism of action of low-dosed LOP is not ovulation inhibition (McCann and Potter, 1994). Ovulation inhibition in women is seen at a dosage of 0.06 mg levonorgestrel /day (Taubert and Kuhl, 1995). Consequently, higher dosages (above the ovulation inhibiting dose in women) block additionally the ovulation and the result is a better contraceptive efficacy. That was the reason for developing a POP using the progestin desogestrel (DSG) in a higher dosage of 0.75 µg/day (Rice et al. 1999). Logically, the contraceptive efficiency calculated on the basis of the so-called Pearl-Index (number of pregnancies per 100 woman years) of the DSG-containing POP is better than that of LOP (0.41 vs. 1.55; see: Benagiano and Primiero 2003). Nevertheless, a relatively high Pearl- Index of 0.41 for a POP using DSG in the dose-range of ovulation inhibition is astonishing and not satisfying. In comparison: In modern COCs a Pearl- Index of 0.1 is recommended (Taubert and Kuhl 1995).

What is the reason, that despite the POP containing DSG in a daily ovulation-inhibiting dosage the contraceptive effectiveness is reduced in comparison to the well-used COCs? In clinical studies, POPs were associated with 12-months pregnancy rates ranging from 1-13 %. Typical-use failure rates are estimated at about 8 - 9 % per year. Because serum progestin levels can be undetectable as early 24 hours after POP ingestion, there is concern that their failure rates may be higher than of combined oral contraception (COCs) due to need for stricter adherence (Burke 2011). Recommendations for specific use of POPs, such as stringent daily timing (POPs should be taken at the same time daily) and missed or late pills rules (backup contraception is recommended for pills taken more than 3 h late !), may also play an important role in clinician's perceptions of POPs and ultimately hinder their provision (Hall et al. 2012).

Additionally, desogestrel is member of a progestin-group with elevated risk of venous venous thromboembolism (VTE). In contrast to this, levonorgestrel is currently the safest progestin (Martinez F et al. 2012). Therefore, the optimization of a levonorgestrel-containing POP is preferable. But an augmentation of the contraceptive effect of a LOP should be include inhibition of ovulation and additionally and mandatory the whole, complete spectrum of pre- as well as post-ovulatory antifertility targets for neutralizing the above discussed failure rates.

From this point of view, the experimental studies show very interesting and unexpected results. Not lower dosages as such for ovulation inhibition are working for the use of all or complete non-ovulatory contraceptive effects. For influencing all the peripheral mechanisms much higher dosages of the progestin levonorgestrel are necessary than for significantly suppressing Luteinizing Hormone (LH) and therefore blocking the ovulation.

For pre-ovulatory contraceptive effects in rabbits an oral dose at least of 5.25 mg levonorgestrel/animal (1.75 mg/kg bodyweight) is necessary (see Examples, Table 1). These effects are independent of the influence of the ovulation itself, because in this animal model the ovulation was induced by exogenous gonadotropins (PMSG and hCG). In comparison to this, a significantly inhibition of physiologic ovulation by levonorgestrel was seen in rabbits with an oral dosage of only 0.5 mg/kg bodyweight (Phillips et al. 1987). Our results show, that higher dosages than for ovulation inhibition are necessary for influencing the complete non-ovulatory, anti-fertility targets.

For post-ovulatory contraceptive effects in rats daily 0.5 mg levonorgestrel/animal (2.5 mg/kg bodyweight subcutaneously) over 4 days are necessary (see see Examples, Table 2). In contrast to this, a significant inhibition of LH-secretion in rats (which is responsible for ovulation) is possible with daily 0.01 mg/animal (0.05 mg/kg body weight subcutaneously). In the same dose-range lies the reduction of the gonadal weight, which is also an indicator for the antigonadotrophic activity of levonorgestrel.

In other words: The dosage for the central antigonadotrophic/antiovulatory effects of levonorgestrel (hypothalamus, pituitary) is in rats 50- times and in rabbits three times lower than that for peripheral contraceptive effects (see Tables 3 and 4). These "inverse" relationships are unexpected for levonorgestrel and non-described for other progestins.

Exceptional in this regard are the observations of Phillips et al. (1987). They found an ovulation-inhibiting dosage for levonorgestel in rats after the very high dosed single oral administration of 128 mg/kg bodyweight. But these findings are not relevant because levonorgestrel and other 19-nor-progestins do not work in rats after oral administration; in contrast to the subcutan (s.c.) administration route (Neumann et al. 1977).

Taken together: Designing of a levonorgestrel-only contraceptive with active ingredient content above the daily ovulation inhibition dose in humans (at least ≥ 0.06 mg/day and woman) shows much better efficiency combining ovulation inhibition as well as peripheral pre-ovulatory and post-ovulatory activities.

The contraceptive activity of this new levonorgestrel-containing POP having ≥ 60 µg levonorgestrel is better than that of the well-known 30 µg-levonorgestrel POP based on our unexpected findings that in higher dosages -beside ovulation inhibition - an additional contraceptive effect (surplus-effect) will be realized by further peripheral contraceptive effects not seen in lower dose levels.

Concerning the exact determination of the human dosage of levonorgestrel there are two valuable orientating and limitative facts. One is the ovulation-inhibiting dose of 0.06 mg/day in cyclic women. Therefore the dosage must be higher than or at least equal to 0.06 mg/day. The other fact is the well-known observation that daily levonorgestrel-dosages over 0.125 mg lead to non-acceptable bleeding irregularities. This is the reason, that in the "classical" oral contraceptives with 0.125 to 0.250 mg levonorgestrel for avoiding bleeding irregularities a combination is necessary with the estrogen ethinylestradiol (from 0.02 to 0.05 mg).Therefore, for designing a levonorgestrel-only contraceptive (without additional estrogen) there is a small window between 0.06 and 0.1 mg/day, preferably between 0.075 and 0.1 mg/day.

The invention is outlined below by means of not-limiting Examples based on animal studies.

### Examples / Experimental Part

### 1. Peripheral Contraceptive Effects

### 1.1. Pre-Ovulatory Administration

Table 1 shows the results of the pre-ovulatory oral administration of levonorgestrel (LNG) over three days in rabbits. On the last day of LNG-administration, the ovulation was "forced" by intravenous (i.v.) injection of human chorionic gonadotropin (hCG). It is shown that this repeated regimen reduced the rate of fertilized eggs in a dose-dependent manner, indicating a fertility-inhibiting effect of pre-ovulatory prolonged LNG administration beyond the ovulation.

### Table 1:

### Influence of repeated oral levonorgestrel (LNG) given pre-ovulatory on the rate of fertilized eggs in New Zealand-rabbits

### Method:

- Day 1:: Induction of Follicle Growth by 50 I.E. PMSG (Pregnant mare's serum gonadotropin) subcutan (s.c.)/Animal
- Days 2 - 4:: Oral Treatment with LNG (per os, p.o.)
- Day 4:: Artificial Insemination; After That Ovulation Induction by 100 I.E. hCG i.v./Animal
- Day 6:: Autopsy ; Counting of *Corpora lutea (or Corpora rubra)* and detection of fertilized and non-fertilized eggs in the tubes and in the uterus.

| **Total Dosage mg/animal p.o. over 3 days** | **n** | **Copora lutea (mean)** | **Total no. of eggs vs. total no. of fertilized (cleaved) eggs** | **%** |
|---|---|---|---|---|
| Control (Sesame Oil) | 32 | 14.4 | 351/318 | 90.6 |
| LNG 0.35 | 9 | 12.6 | 92/78 | 84.8 |
| LNG 1.75 | 9 | 10.6 | 81 /49 | 60.5 |
| LNG 5.25 | 8 | 14.8 | 112/61 * | 54.5 |

| | | | | |
|---|---|---|---|---|
| *= Significantly different to control (p< 0.05). | | | | |

### 1.2. Post-Ovulatory Administration

In rats, the oral bioavailability of progestins is poor, Therefore, levonorgestrel must be given parenterally (e.g. in contrast to rabbits).

Table 2 shows the postovulatory contraceptive activities of LNG given to paired female rats. Whereas 20 mg/kg bodyweight (b.w.). given once post-coitally (immediately after mating) was ineffective, the prolonged administration of 5mg/kg b.w. and day over 4 days was fully effective.

### Table 2:

### Influence of subcutaneous (s.c.) levonorgestrel (LNG) given post-ovulatory on fertility of female paired Wistar-rats.

### Method:

- Day 1:: Detection of sperms in the vagina
- Day 10:: Autopsy and macroscopic inspection of the uteri

| Mode | Total dose mg /animal /4 days | No. of treated animals vs. no. of intact pregnancies | Pregnancy inhibition (%) |
|---|---|---|---|
| Sesame Oil s.c. days 1-4 post insemination (p.i.) | - | 12/12 | 0 |
| LNG s.c. once Day 1 p.i. | 4.0 | 12/12 | 0 |
| LNG s.c. days 1-4 p.i. | 0.2 | 10/9 | 10 |
| LNG s.c. days 1-4 p.i. | 2.0 | 10/0 | 100 |

### 2. Central Contraceptive (Anti-ovulatory) Effects

The influence of levonorgestrel on the secretion of gonadotrophins from the pituitary can be estimated by recording the dose-effect relationships in reducing the weight of the gonads of immature male rats. The results are shown in Table 3.

**Table 3:**

| **The Influence of levonorgestrel given daily subcutaneously over 14 days on the weight of gonads in immature male Wistar-rats.** | | | |
|---|---|---|---|
| Group | Total dose mg/animal over 14 d | N | Gonadal weight/mg Mean ± (S.E.M.) |
| Starting control | - | 10 | 216.9 ± 11,0 |
| Final control | - | 9 | 873.5 ± 62.5 |
| levonorgestrel | 0.14 | 10 | 579.7 ± 44.7 * |
| levonorgestrel | 0.42 | 10 | 385.7 ± 74.3 * |
| levonorgestrel | 1.40 | 10 | 180.8 ± 105.5 * |

| | | | |
|---|---|---|---|
| *= Significantly different to final control (p< 0.05). | | | |

The direct effect of levonorgestrel on the secretion of the pituitary Luteinizing Hormone (LH) is shown in Table 4.

**Table 4:**

| **The influence of levonorgestrel given daily subcutaneously on the serum-levels of Luteinizing Hormone (LH) in male, castrated Wistar-rats (estimated by radioimmunoassay)** | | |
|---|---|---|
| Group | Total dose mg/animal/14d | Luteinizing Hormone ng/ mL serum |
| Control, intact (n= 8) | - | 4.9 ± 1.0 |
| Control, gonadectomized (n=6) | - | 127.0 ± 11.0 |
| levonorgestrel (n = 5) | 0.14 | 68.4 ± 15.4 * |
| levonorgestrel (n = 7) | 1.40 | 2.2 ± 0.3 * |

| | | |
|---|---|---|
| *= Significantly different to gonadectomized control (p< 0.05). | | |

Regarding the use in human females a tablet/coated pill was prepared which contains 0.075 mg levonorgestrel. Further pharmaceutical acceptable carriers and excipients contained in the tablet/coated pill are talcum, magnesium stearate, magnesium carbonate, calcium carbonate, E123, wax and carnauba wax. The tablet was taken by fertile women every day without a break, i.e. every day of their menstruation cycle and with no break between cycles.

Additionally, a soft-gelatine capsule was prepared which contains 0.100 mg levonorgestrel. Further pharmaceutical acceptable carriers and excipients contained in the capsule are peanut oil, titanium dioxide (E 171), gelatine, glycerol, and lecithin. The capsule was taken by fertile women every day without a break, i.e. every day of their menstruation cycle and with no break between cycles.

### References Cited:

- Benagiano G, Primiero FM. Seventy-five microgram desogestrel minipill. A new perspective in estrogen-free contraception. Ann NY Acad Sci 2003: 997: 263-173.
- Burke AE. The state of hormonal contraception today: benefits and risks of hormonal contraceptives:progestin-only contraceptives. Am J Obstet Gynecol 2011; 205 (4 Suppl):S14-17.
- Cackovic M, Paidas MJ. Should oral contraceptives be sold over-the-counter? No. Contemporary OB/GYN 2008; 53:63-74.
- Cameron C, Trenor III, Chung RJ, Michelson AD, Neufeld EJ, Gordon CM, Laufer MR, Emans SJ. Hormonal contraception and thrombotic risk: A multidisciplinary approach. Pediatrics 2011; 127: 347-357.
- Centers for Disease Control and Prevention. U.S. medical eligibility criteria for contraceptive use. Morb Mortal Wkly Rep 2010; 59: 1-86.
- Grossman D, Fernandez L, Hopkins K, et al. Accuracy of self-screening for contraindications to combined oral contraceptive use. Obstet Gynecol 2008; 112: 572-578.
- Grossman D, White K, Hopkins K, et al. Contraindications to combined oral contraceptives among over-the-counter and clinic pill users. Obstet Gynecol 2011; 17:558-565.
- Hall KS, Trussell J, Schwarz EB. Progestin-only contraceptive pill use among women in the United States. Contraception 2012 Jun 6 (Epub ahead of print) doi:10.1016/j.contraception.2012.05.003.
- Martinez F, Ramirez I, Pérez-Campos E, Latorre K, Lete I. Venous and pulmonary thromboembolism and combined hormonal contraceptives. Systematic review and meta-analysis. Eur J Contracept Reprod Health Care 2012; 17: 7-29.
- McCann MF, Potter LS. Progestin-only contraception: a comprehensive review. Contraception 1994; 50 (Suppl. 1): S13-S21.
- Neumann F, Elger W, Nishino Y, Steinbeck H. Problems of dose finding: sexual hormones. Arzneimittelforschung/Drug Research 1977; 27 (2A): 296-318.
- Oxford/FPA. Study report. Vessey M, Lawless M, Yeates D. Efficacy of different contraceptive methods. Lancet 1982; 10: 841-842.
- Phillips A, Hahn DW, Klimek S, McGuire JL. A comparison of the potencies and activities of progestogens used in contraceptives. Contraception 1987; 36: 181-192.
- Rice CF, Killick SR, Dieben T, Coelingh Bennink H. A comparison of the inhibition of ovulation achieved by desogestrel 75 µg and levonorgestrel 30 µg daily. Hum Reprod 1999;14:982-985.
- Shortridge E, Miller K. Contraindications to oral contraceptive use among women in the United States, 1999-2001. Contraception 2007; 75:355-360.
- Sitruk-Ware R. New progestagens for contraceptive use. Hum Reprod Update 2006: 12: 169-178.
- Taubert H-D, Kuhl H. Kontrazeption mit Hormonen (2nd edition).Georg Thieme Verlag Stuttgart, New York 1995;page 84.
- White K, Potter JE, Hopkins K, Fernandez L, Amastae J, Grossman D. Contraindications to progestin-only oral contraceptive pills among reproductive-aged women. Contraception; doi:10.1016/j.contraception.2012.01.008.
- World Health Organization Collaborative Study of Cardiovascular Disease and Steroid Hormone Contraception. Cardiovascular disease and use of oral and injectable progestin-only contraceptives and combined injectable contraceptives:results of an international, multicenter, case-control study. Contraception 1998;57:315-324.

## Claims

1. Pharmaceutical composition for oral contraception for a woman of fertile age consisting of 60 to 100 µg of levonorgestrel and one or more pharmaceutical acceptable carriers and excipients.

2. Pharmaceutical preparation for oral contraception consisting of a number of separately packed and individually removable daily dosage units intended for consecutive daily oral administration, each dosage unit consisting of 60 to 100 µg of levonorgestrel and one or more pharmaceutical acceptable carriers and excipients.

3. Pharmaceutical preparation for oral contraception according to claim 2, **characterized by** containing at least 28 separately packed and individually removable dosage units intended for consecutive daily oral administration for a period of at least 28 consecutive days.

4. Use of an oral dosage form consisting of 60 to 100 µg of levonorgestrel and one or more pharmaceutical acceptable carriers and excipients per dosage unit for oral contraception for a woman of fertile age by administering one dosage unit daily during the complete menstruation cycle.
